# EUROPEAN PATENT APPLICATION

(11) **EP 0 555 927 A1**
(43) Date of publication of application: **18.08.1993**
(21) Application number: 93200368.4
(22) Date of filing: 11.02.1993
(51) Int. Cl.: A61M 39/00

(54) **Connecting element and pocket provided with such an element**

(30) Priority: 14.02.1992 IT SO920003 U
(71) Applicant: MARGI S.r.L., I-23100 Sondrio (IT)
(72) Inventor: Giacomelli, Diego, I-23100 Sondrio (IT)
(74) Representative: Faraggiana, Vittorio, Dr. Ing.

(57) **Abstract**

A connecting element (13) for fluids in the medical field, comprises an inner connecting passage (14) opening, through a coupling outlet, at a head face (15) of the connecting element. The coupling outlet extends inside the passage with a conical side surface (16) tapering inwardly to form a female coupling for a corresponding male fitting piece (30). The coupling outlet is closed by a plug (24) having a generally tubular end (23) sealingly fitted in a mating seating (22) formed by a radial enlargement of the coupling outlet. The plug extends externally of the head face (15) with a gripping projection (25) and has a frangible line (28) between the gripping projection and tubular end for separation by breaking of the two portions, in order to open the coupling outlet by removal of the gripping projection (25). The connecting element can be advantageously inserted in the end portion of a pipe (11) issuing from a fluid-containing envelope so as to form a pocket closed by said pipe union.

## Description

The present invention relates to a connecting element or pipe union, and a fluid-containing pocket provided with said element, of the type used in the medical field and designed to contain and deliver physiological salt solutions for example.

Several different types of pipe unions are known in the medical field for mutually connecting pipings and accessory elements such as taps, flow regulators, extension pipes, etc, for delivery and processing of fluids.

The simplest of them comprises a tubular element provided with two shanks at the opposite ends thereof, to be fitted between a pipe issuing from a pocket and a union or extension pipe.

As described in the Italian patent for industrial utility model No. 0 216 302, the male connection can be supplied with an occluded tip which is a one-piece extension of the generally conical part of the connection. By providing an appropriate frangible line, the connection can be easily opened by merely bending the tip which thus separates from the remainder.

A different type of pipe union is made from a tubular element provided at one end with a female connection into which flow-controlling taps, extension pipes, etc. can be inserted. The pipe union also comprises a screw thread or outer engaging wings for screw-locking the elements fitted therein. This type of connection is generally referred to as "Luer-lock". The above pipe union offers a more flexible use than the preceding one.

The tubular element is closed by a plug to be screwed down externally of the Luer-lock connection and to be untightened when the connection is to be used. A fitting shank for a pipe issuing from a pocket for example, may be provided at the other end of the pipe union, in the same manner as in the preceding pipe union.

This type of embodiment, although universally adopted, involves some problems in connection with the sterilization of the space inside the connecting element. In fact, if the sterilization is carried out before mounting of the plug a contamination is likely to occur in the lapse of time between sterilization and mounting of the plug. If the sterilization is carried out after mounting of the plug, the plug is likely to become locked by the action of heat produced during the sterilization, which will make the subsequent disassembling impossible or at all events difficult.

Even if the above drawbacks should not occur, an accidental contamination is always possible after the pocket has been produced and filled, since subsequent disassemblings and reassemblings of the plug before the pocket reaches the final user are not prevented.

A general object of the present invention is to eliminate the above drawbacks, by providing a pipe union equipped with a Luer-lock connection having an improved closing plug enabling a quick and safe opening and preventing contamination of the inside of the pipe union.

In view of the above object, in accordance with the invention a connecting element for fluids in the medical field has been devised which comprises an inner connecting passage opening, through a coupling outlet, at a head face of the connecting element, the coupling outlet extending further within the passage by a conical side surface tapering inwardly so as to form a female coupling for a corresponding male fitting piece the coupling outlet being occluded by a closing element which is removable for insertion of said male fitting piece thereinto, characterized in that the removable closing element is formed with a plug having a generally tubular end sealingly fitted in a mating seating consisting of a radial enlargement of the coupling outlet, the plug extending externally of the head face by a gripping projection and having a frangible line between the gripping projection and tubular end for separation by breaking of the two portions in order to open the coupling outlet by removal of the gripping projection.

Such a pipe union can be advantageously employed in making a pocket comprising a flexible fluid-containing envelope, from which a pipe provided with a free end terminating with the above pipe union projects.

For better clarifying the innovatory principles of the present invention and the advantages of same as compared to the known art, a possible embodiment of said invention applying the above principles will be given hereinafter, by way of non-limiting example, with reference to the accompanying drawings, in which:
- Fig. 1 is a part sectional exploded view of a pocket provided with a connecting element made in accordance with the invention;
- Fig. 2 shows the end of the pipe union seen in Fig. 1 in an assembled condition;
- Fig. 3 shows the end of the pipe union seen in Fig. 1 in a first opening step of same;
- Fig. 4 is a view of the pocket seen in Fig. 1 showing the pipe union in an open condition and connected to an accessory device;
- Fig. 5 is an end view of the opened pipe union.

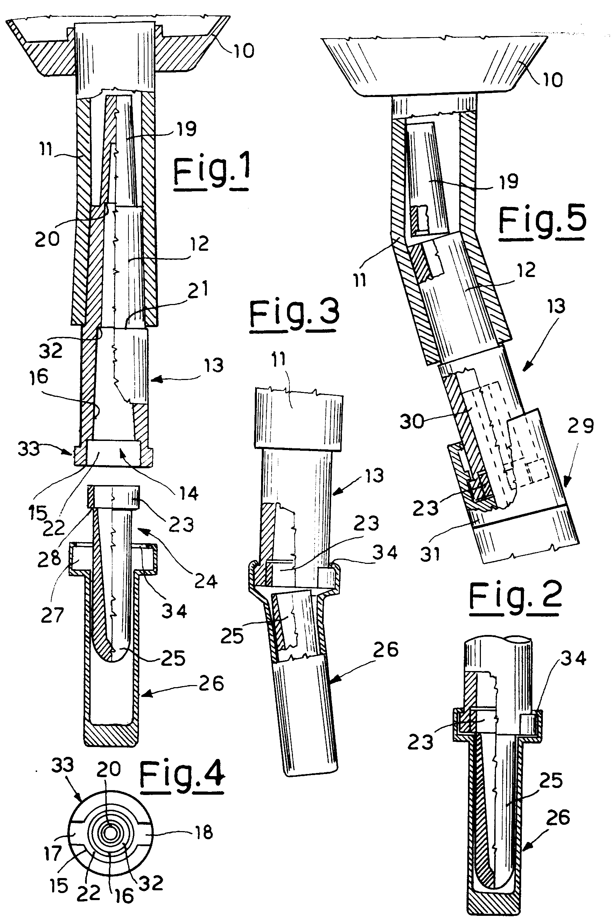

Referring to the drawings, as viewed from Fig. 1, a connecting element 13 has an inner axial passage 14 opening, through a free end, at a head face 15 having a Luer-lock type connection 15. In particular, the Luer-lock pipe union comprises a surface 16 extending from the head face towards the inside of the axial passage 14. The surface 16 is of conical form tapering towards the inside of the pipe union so as to provide a female snap-fitting piece for fitting shanks belonging to devices to be connected, as better clarified in the following.

Externally of the coupling outlet of the pipe union, provision is made for screw coupling means 33 the thread of which matches with the thread in corresponding screw means provided on the device to be connected. The thread can consist of a complete helix or several separate segments. It may even be defined by two radial wings 17, 18 alone, located at opposite positions, as clearly shown in Fig. 4. Thus the wing pair enables a bayonet coupling to be accomplished.

In accordance with the innovatory principles herein claimed, the opening of the inner passage 14 has a recessed initial section so as to form a seating 22 which is a radial enlargement of the coupling outlet. A closing plug 24 has a tubular fitting end or ring 23 matching the shape of the seating 22 so that it can be sealingly received thereinto. The plug extends from the fitting end 23 towards the outside of the pipe union with an internally hollow gripping projection 25 which is closed at its free end. A reduced-thickness portion or frangible line 28 which is coincident with the coupling outlet of passage 14, is provided on the plug between the gripping projection and fitting end, for mutual separation by bending.

To this end the plug is made of frangible plastic material of relatively high stiffness.

Optionally, a protection cap 26 can be further provided of a shape adapted to receive the plug and exhibiting an enlarged section or opening 27 for radially embracing the end of the Luer-lock pipe union at the screw coupling means. As will be best understood in the following, the cap can be made of relatively yielding and flexible plastic material.

The connecting element 13 can comprise, in an opposite direction to the outlet 15, an end shank or fitting male piece 12 over which a pipe 11 for connection with the inner passage 14 can be fitted. A connecting step 21 can form an abutment preventing the excessive introduction of the connecting element 13 into the pipe 11. In this case the conical surface 16 can terminate towards the inner part of the pipe union with a step 32 close to the outer step 21, in order to keep the wall thickness substantially constant.

The fitting shank 12 can terminate with a closed end 19 having a frangible portion at its jonction point 20 with the fitting shank so as to enable separation between the closed end and the shank by mere bending. To this end, the fitting shank 12 is made of frangible plastic material of relatively high stiffness, and advantageously the same material as the element 13, as will be clear to a person having ordinarny skill in the art.

The pipe 11 can advantageously protrude from a flexible envelope 10 (only partly shown as known in the art and therefore easily conceivable by a technician), so as to make a fluid-containing pocket for use in the medical field. The short delivery pipe 11 can be of one piece construction with the pocket or connected to the pocket by gluing, melting, molding, etc.

Shown in Fig. 2 is the end of the connecting element when it is completely assembled. The plug is sealingly inserted in the complementary seating 22, fastened thereto by sticking for example, thermal action or others, so that it is substantially steadfast. The thickness of the annular wall of the fitting end 23 of the plug is equal to or smaller than the height of the annular seating 22 so as to reduce the passage towards the conical surface 16.

A protection cap 26 can be fitted over the plug, which cap can have an inward radial rim 34 to be adapted to the connection screw means 17, 18 and receive them. The cap embodies a sealing means capable of avoiding contaminations of the fitting means and the pipe union opening at the outside of the plug.

The connecting element can be sterilized using usual techniques, such as exposure to gamma rays, as well-known in the art. Since the plug must be fixed in a steady manner, sterilization can be advantageously executed after assembling of the parts, due to the fact that, in opposition to the known art, the risk of causing jammings does not exist.

Internal recontamination is also efficiently prevented by the plug 25, in that it cannot be removed and reassembled in an indiscriminate manner as it happened with screw plugs of the known art.

The opening step of the pipe union is shown in Fig. 3. It is sufficient to bend the projecting plug end, even without removing the cap which to this end is made of a flexibile material, for achieving the separation of the projection 25 from the fitting ring 23 along the frangible line 28. The ring 23 is trapped in its seating whereas the projection 25 is contained in the cap that can be removed by a mere pulling action by virtue of its yielding nature.

As shown in Fig. 5, to the Luer-lock connecting element thus opened any appropriate accessory element 29 of the known art can be locked in the usual manner, such as a shutoff tap, a flow regulator, an end piece of a cannula, etc.. The conical surface 16 will receive by snap fitting the mating male piece 30 of the connected member, whereas the screw means will lock a corresponding outer ring nut 31 for screw fastening of the element 29 in a mechanical manner. The presence of the ring 23 which is the residual fixed part of the plug will not interfere with the male fitting piece 30 as it is of a reduced thickness so that it does not protrude in the pipe, and is located in the initial opening area.

If the rear shank 19 is provided, the pipe 11 can be brought into fluid communication with the Luer-lock pipe union by bending of the pipe 11 so as to break the shank end portion 19 along its frangible line.

The presence of the further closure embodied by the shank 19 can advantageously prevent sudden liquid leakages from the pocket when the plug 24 is broken. Obviously the shank can be omitted if said accessory function is not of interest. The presence of the shank also makes it possible to sterilize the coupling element 13 internally before its mounting to the pipe without the risk of subsequent contaminations.

At this point it is apparent that the intended purposes are achieved.

It is understood that the foregoing description has been given purely as an example applying the innovatory principles of the invention and therefore must not be interpreted as a limitation of the true scope of this invention as claimed in the following claims.

For example, the proportions of the different elements may vary depending on practical requirements. In addition the pipe 11 can be of any length.

## Claims

1. A connecting element (13) for fluids in the medical field, comprising an inner connecting passage (14) opening, through a coupling outlet, at a head face (15) of the connecting element, the coupling outlet extending further within the passage by a conical side surface (16) tapering inwardly so as to form a female coupling for a corresponding male fitting piece (30), the coupling outlet being occluded by a closing element which is removable for insertion of said male fitting piece thereinto, characterized in that the removable closing element is formed with a plug (24) having a generally tubular end (23) sealingly fitted in a mating seating (22) consisting of a radial enlargement of the coupling outlet, the plug extending externally of the head face (15) with a gripping projection (25), and having a frangible line (28) between the gripping projection and tubular end for separation by breaking of the two portions, in order to open the coupling outlet by removal of the gripping projection.

2. A connecting element according to claim 1, characterized in that radial locking means (33) is present around the coupling outlet for receiving mating locking means connected to the male fitting piece.

3. A connecting element according to claim 2, characterized in that the radial locking means comprises a thread (33) for screwing of the locking means connected to the fitting piece.

4. A connecting element according to claim 2, characterized in that the radial locking means comprises diametrically opposite projecting wings (17, 18).

5. A connecting element according to claim 1, characterized in that a protection cap (26) is fitted over the gripping projection (25) of the plug, the opening of said cap radially embracing the connecting element in the vicinity of the outlet thereof.

6. A connecting element according to claim 5, characterized in that the protection cap (26) is made of flexible material so as to enable bending of same for breaking of the gripping end (25) contained therein.

7. A connecting element according to claim 1, characterized in that the inner passage (14) terminates with a shank (12) extending on the opposite side with respect to the coupling outlet and the end portion of a pipe (11) can be fitted over said shank.

8. A connecting element according to claim 7, characterized in that the shank (12) has a closed end (19) to be broken by bending along a frangible line (20) for opening of a passage therethrough.

9. A connecting element according to claims 2 and 5, characterized in that the cap radially embraces the connecting element as far as it covers the radial locking means.

10. A fluid containing pocket for use in the medical field, comprising a flexible fluid-containing envelope from which a pipe (11) projects the end of which terminates with a connecting element (13) embodied in accordance with the preceding claims.
